Europäisches Patentamt

European Patent Office

Office européen des brevets

(19)

(11) EP 1 396 229 A1

(12) EUROPEAN PATENT APPLICATION
published in accordance with Art. 158(3) EPC

(43) Date of publication:
10.03.2004 Bulletin 2004/11

(51) Int Cl.⁷: A61B 6/03

(21) Application number: 02730795.8

(22) Date of filing: 30.05.2002

(86) International application number:
PCT/JP2002/005284

(87) International publication number:
WO 2002/098293 (12.12.2002 Gazette 2002/50)

(84) Designated Contracting States:
AT BE CH CY DE DK ES FI FR GB GR IE IT LI LU
MC NL PT SE TR

(30) Priority: 01.06.2001 JP 2001166082

(71) Applicant: Hitachi Medical Corporation
Tokyo 101-0047 (JP)

(72) Inventors:
• OOTA, Shirou
  Matsudo-shi, Chiba 270-2251 (JP)
• TSUKIZU, Takashi
  Nagareyama-shi, Chiba 277-0102 (JP)

(74) Representative: Strehl Schübel-Hopf & Partner
Maximilianstrasse 54
80538 München (DE)

(54) X-RAY CT DEVICE, IMAGE PROCESSOR, AND METHOD FOR PROCESSING IMAGE OF X-RAY CT DEVICE

(57) An X-ray CT device including: an X-ray source; a detector for detecting X-rays from the X-ray source and transmitted through a subject to be examined; an image reconstruction unit for generating X-ray data detected by the detector to generate a tomographic image of the subject; a display unit for displaying the tomographic image; a memory for storing data obtained by the X-ray detector and the tomographic image data; an input unit for inputting data of tomographic scanning conditions of the subject; and an image processing unit for calculating an X-ray repetition rate, an X-ray intensity and an X-ray irradiation range in the subject based on the inputted data of scanning conditions, and generating image data for displaying a value of at least one of the X-ray repetition rate, the X-ray intensity and the X-ray irradiation range as a graphic image on the display unit based on the calculation result.

FIG. 1

ENLARGED VIEW

10'  11  12

EP 1 396 229 A1

## Description

Technical Field

The present invention relates to a technique applicable to a medical diagnostic apparatus using X-rays, and particularly relates to a technique effective in application to a technique for setting an X-ray irradiation range, an X-ray irradiation width, an X-ray intensity, an X-ray dose to be radiated repeatedly to a location of a subject to be examined (hereinafter referred to as "X-ray repetition rate") and other scanning conditions of the device in an X-ray CT device.

Background Art

The X-ray repetition rate and the X-ray intensity are significant factors in determining the quality of an image obtained by image reconstruction of an X-ray CT device. When the X-ray repetition rate and the X-ray intensity are set at large values, a high-quality image can be obtained, but the exposed dose to a subject to be examined becomes a problem on the other hand.

An operator sets the X-ray repetition rate and the X-ray intensity while taking antithetic items such as the image quality and the exposed dose to the subject into consideration.

However, in the prior art, an operator has estimated the X-ray repetition rate and the X-ray intensity from his/her experience. When a location of a subject to be scanned is determined, the operator estimates the X-ray repetition rate (X-ray irradiation dose) and the irradiation range in CT image scanning experientially on the basis of the slice thickness and the feed pitch of a table on which the subject lies. In a similar manner thereto, the operator also estimates the X-ray intensity from the time per scan and the current value and voltage value of an X-ray tube. When experientially determining proper values of the X-ray repetition rate and the X-ray intensity in consideration of the required quality level of a CT image, the physique of the subject, and the like, the operator estimates the scanning conditions for obtaining these values, i.e., the slice thickness, the table feed pitch, the scan time, the current value and the voltage value, and again sets the estimated values as final scanning conditions. A CT image of the subject is actually taken under the set scanning conditions. Accordingly, in this conventional method, it is not easy to set proper scanning conditions satisfying a required image quality and a minimum X-ray exposed dose because the scanning conditions are determined depending on the operator's experience.

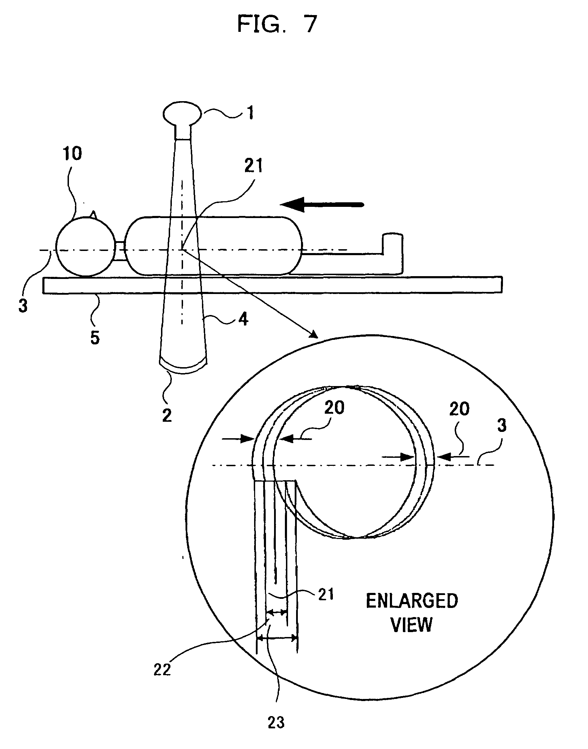

A method for producing a CT image in a helical scanning X-ray CT device will be described with reference to Fig. 7. In the helical scanning X-ray CT device, an X ray tube 1 and a detector 2 are disposed to be opposed to each other through a movable table 5 on which a subject 10 to be examined lies. The detector 2 detects X-rays 4 radiated from the X-ray tube 1 and transmitted through the subject 10. The set of the X-ray tube 1 and the detector 2 is called a scanner. The scanner can revolve continuously around the body axis 3 of the subject 10. The subject 10 is irradiated with X-rays while the scanner is revolved. When the table 5 is moved in an arrow direction (body axis direction) at the same time, CT image data based on a helical scan can be obtained.

In order to obtain a CT image on a broken line 21 (slice position) of the body of the subject 10 in this helical scanning CT device, the slice position 21 must be always irradiated during one revolution of the scanner while the table 5 is moved. An X-ray irradiated state near the body axis in the slice position 21 of the subject 10 during this helical scanning is shown in the enlarged view of Fig. 7. In the enlarged view, the X-ray irradiated portion is shown in a twisted belt-like shape. The X-ray irradiation area to the subject 10 is shown as a belt twisted and once turned around the body axis 3 within an X-ray irradiation width 20. At one scan, a range represented by the reference numeral 23 is irradiated with X-rays. Thus, a tomographic image of the slice position 21 with a slice thickness 22 can be reconstructed.

In the conventional helical scanning CT device, although the slice position 21 can be confirmed on a scanogram, it is not easy for the operator to grasp the slice position 21 corresponding to the image reconstruction position, the X-ray irradiation width 20, the X-ray irradiation range 23, the X-ray intensity and the interrelationship among them instantly as shown in the enlarged view of Fig. 7. Accordingly, it is impossible to easily judge how much X-ray dose to the subject 10 will reach, whereby the X-ray dose and/or the irradiation range is determined from experience or by making complicated calculations for setting the scanning conditions.

It is an object of the present invention to provide an X-ray CT device and an image processing method by which an operator can judge visually and easily the X-ray repetition rate, the X-ray intensity and the X-ray irradiation range so that the operator can determine proper values of the X-ray repetition rate, the X-ray intensity and the X-ray irradiation range in order to set scanning conditions required for satisfying a desirable image quality and a minimum exposed dose.

The other objects, features and advantages of the present invention will be made clear from the following description of the embodiment of the present invention with reference to the accompanying drawings.

Disclosure of the Invention

An X-ray CT device according to the present invention includes: an X-ray source; a detector for detecting X-rays radiated from the X-ray source and transmitted through a subject to be examined; an image reconstruction unit for processing X-ray data detected by the detector to generate a tomographic image of the subject; a display unit for displaying the tomographic image; a memory unit for storing the data obtained by the X-ray detector and data of the tomographic image; an input unit for inputting data of tomographic scanning conditions of the subject; and an image processor for calculating an X-ray repetition rate, an X-ray intensity and an X-ray irradiation range in the subject based on the inputted data of scanning conditions, and generating image data for displaying at least a value of any one of the X-ray repetition rate, the X-ray intensity and the X-ray irradiation range as a graphic image on the display unit based on a result of the calculation.

An image processor of an X-ray CT device according to the present invention includes: an input unit for inputting data obtained by detecting X-rays radiated from an X-ray source and transmitted through a subject to be examined, and data of tomographic scanning conditions of the subject; a memory unit for storing data of the tomographic image; and an image processing portion for calculating an X-ray repetition rate, an X-ray intensity and an X-ray irradiation range in the subject based on the inputted data of scanning conditions, and generating image data for displaying at least a value of any one of the X-ray repetition rate, the X-ray intensity and the X-ray irradiation range as a graphic image on a display unit based on a result of the calculation.

An image processing method for making an X-ray CT device generate an image of a subject to be examined according to the present invention includes the steps of: generating a scanogram of the subject by the X-ray CT device; receiving data of scanning conditions specified for obtaining a tomographic image of the subject; calculating an X-ray repetition rate, an X-ray intensity and an X-ray irradiation range in the subject based on the inputted data of scanning conditions; generating image data for displaying at least a value of any one of the X-ray repetition rate, the X-ray intensity and the X-ray irradiation range as a graphic image on a display unit based on a result of the calculation; displaying the scanogram and the graphic image superimposed on each other on the display unit; determining and inputting tomographic scanning conditions of the subject based on the image displayed on the display unit; and performing tomographic scan upon the subject using the X-ray CT device based on the determined scanning conditions.

According to an embodiment of the present invention, the X-ray repetition rate, the X-ray intensity and the X-ray irradiation range are expressed by belt-like lines, and the X-ray repetition rate at the time of scanning corresponding to an X-ray thickness (X-ray irradiation width) and a table transferring rate is calculated automatically and displayed as a graphic image.

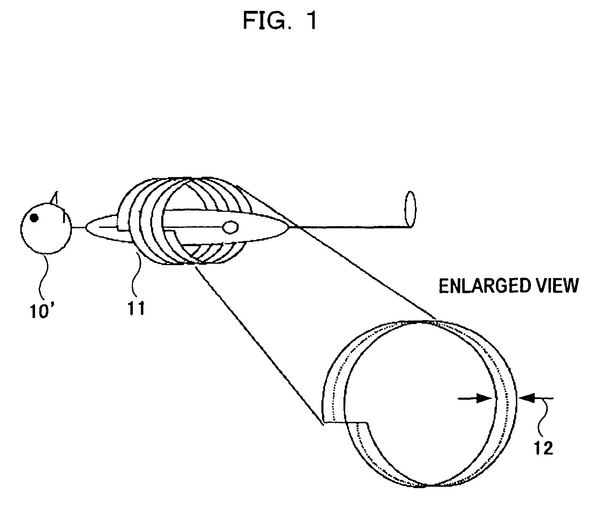

In addition, if necessary, belt-like lines (the reference numeral 11 in Fig. 1) may be displayed on a scanogram image of the subject or a diagram imitative of a human body (the reference numeral 10' in Fig. 1).

On this occasion, the belt-like line thickness (the reference numeral 12 in Fig. 1) is displayed in proportion to the X-ray thickness (X-ray irradiation width) and the size of the scanogram image (the reference numeral 10' in Fig. 1).

The display color and contrast of the belt-like image is displayed in correspondence with the tube current and/or the tube voltage so that the X-ray intensity can be identified visually. When an image reconstruction position is set, the X-ray irradiation range corresponding to the image reconstruction position is displayed automatically. These settings can be done through a keyboard or another input device while the belt-like image in Fig. 1 is confirmed on the screen.

Brief Description of the Drawings

Fig. 1 is a view showing the concept of display of an X-ray irradiation range in an embodiment of an X-ray CT device according to the present invention;
Fig. 2 is a block diagram showing the configuration of an image processor in the embodiment of the X-ray CT device according to the present invention;
Fig. 3 is a view for explaining a change of the X-ray irradiation range in the embodiment of the X-ray CT device according to the present invention;
Fig. 4 is a view for explaining a change of the X-ray irradiation width in the embodiment of the X-ray CT device according to the present invention;
Fig. 5 is a view for explaining a change of a table feed pitch in the embodiment of the X-ray CT device according to the present invention;
Fig. 6 is a view for explaining a change of an X-ray intensity in this embodiment;
Fig. 7 is a view for explaining a method of image reconstruction in a helical scanning X-ray CT device;
Fig. 8 shows another example of a graphic image indicating the X-ray irradiation range; and
Fig. 9 is a flow chart showing the procedure for generating a CT image in the X-ray CT device according to the present invention.

Best Mode for Carrying out the Invention

Hereinbelow, the present invention will be described in detail in reference of the embodiment and the drawings.

Incidentally, in all the drawings for explaining the embodiment of the invention, parts having one and the same function are denoted by the same reference numerals correspondingly, and repeated description thereof will be omitted.

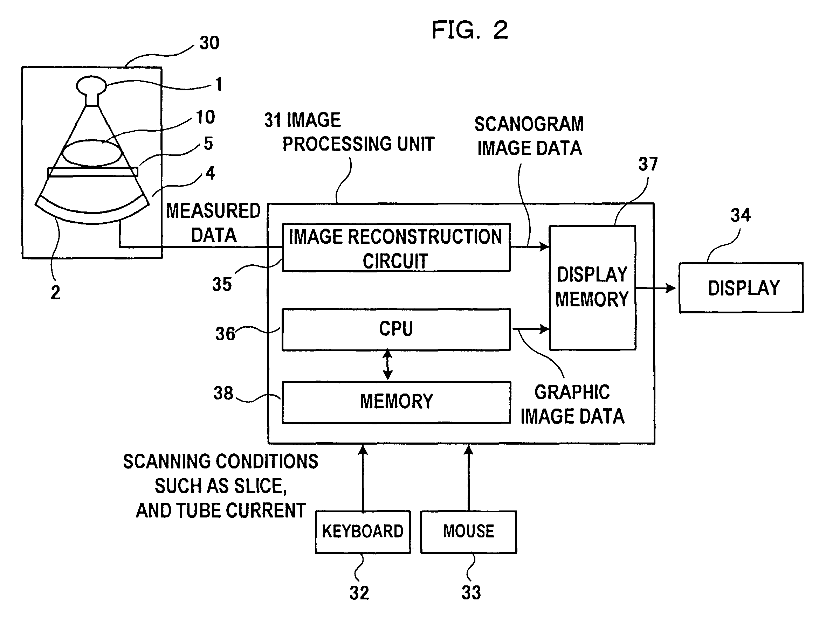

As shown in Fig. 2, an X-ray CT device according to the present invention includes a scanner 30 having an X-ray tube 1 and an X-ray detector 2, disposed opposite to each other, a table 5 for mounting a subject 10 on it and between the X-ray tube 1 and the X-ray detector 2 and moving the subject 10, a scanner control unit (not shown) for controlling the operation of the scanner 30 and the operation of the table 5, an X-ray control unit (not shown) for controlling the generation of X-rays, an image processing unit 31 for revolving the X-ray tube 1 and the X-ray detector 2 around the subject while radiating X-rays thereto, and for processing data obtained from the X-ray detector 2 to reconstruct an image, a display unit 34 for displaying the reconstructed image, a display memory 37 for storing raw data obtained from the X-ray detector or an image obtained therefrom by calculation, a CPU 36 for totally controlling the respective units, and input units 32 and 33 for inputting various scanning conditions described above. As the fundamental configuration of this X-ray CT device, the device disclosed in the specification of U.S. Patent Application serial No. 09/823,461 filed on March 30, 2001 by the present applicant can be adopted. The contents disclosed in U.S. Patent Application serial No. 09/823,461 is cited and incorporated into this application. Accordingly, only essential constituent parts of the present invention will be illustrated and described, and the scanner control unit and the X-ray control unit will not be illustrated and described.

According to an embodiment of the present invention, an X-ray irradiation range, an X-ray irradiation width, an X-ray intensity, an X-ray dose radiated repeatedly to a location of the subject (hereinafter referred to as "X-ray repetition rate"), and other scanning conditions of the device are set in the aforementioned X-ray CT device.

Fig. 1 is a view showing an example of a displaying mode when the X-ray irradiation range of the helical scanning X-ray CT device according to the embodiment of the present invention is displayed on the display 34. The reference numeral 10' represents a model image or a scanogram image of the subject 10; the numerical reference 11 represents an X-ray irradiation range; and the numerical reference 12 represents an X-ray irradiation width.

On the screen of the display 34, the X-ray irradiation range 11 is displayed on the scanogram image of the subject as a spiral belt-like graphic image. The image of the X-ray irradiation range 11 is superimposed on the model image 10' or the scanogram of the subject 10 to be in agreement with their slice positions. The drawing in the right lower of Fig. 1 is an enlarged view of an imaginary view of an irradiation range corresponding to one revolution of scan is extracted from the X-ray irradiation range 11 of the graphic image. An operator can confirm the X-ray irradiation range when glancing at the image of Fig. 1 displayed on the monitor.

Next, description will be made of an image processing method for generating the graphic image of the X-ray irradiation range as shown in Fig. 1, and displaying the graphic image while superimposed on the model image or the scanogram of the subject.

Fig. 2 is a block diagram of the embodiment of the X-ray CT device having the image processing unit 31 for generating data of a display image as shown in Fig. 1.

In the X-ray CT device according to this embodiment, the image of Fig. 1 corresponding to initially set X-ray conditions or an initially set image reconstruction position is displayed on the display 34 as shown in Fig. 2. Viewing this image, the operator uses an input unit such as the keyboard 32, the mouse 33 or the like to input X-ray scanning conditions such as a slice thickness, a feed pitch of the table 5, an image reconstruction position and the like.

From the inputted X-ray scanning conditions, the X-ray irradiation range, the X-ray repetition rate and the X-ray intensity are automatically and immediately calculated in the image processor 31. The X-ray irradiation range may be designated through the input unit in advance by the operator using positioned information of the body of the subject or obtained by calculating a requisite range for reconstructing the image upon designation of a slice position and the number of slices through the input unit. The X-ray repetition rate is calculated by the CPU 36 according to the following expression (1).

$$\text{repetition rate (\%)} = \text{slice thickness} \times$$

$$\text{simultaneously measured slice number} \times \text{index}$$

$$\text{pitch} \qquad (1)$$

The X-ray intensity is calculated according to the following expression (2).

$$\text{X-ray intensity} = \text{mAs value} \times \text{repetition rate}$$

$$\times \text{CTDIw value} \qquad (2)$$

$$\text{mAs} = \text{X-ray tube current} \times \text{scan time}$$

The CTDIw values are specific for systems and uniquely determined by the mAs values. The index pitch is a moving distance of the table at each revolution of the scanner. Next, images are processed based on the X-ray irradiation range, the X-ray repetition rate and the X-ray intensity, automatically calculated in advance in the image processor and displayed as belt-like lines 11 (Fig. 1) on the display 34. Graphic image data of a basic standard spiral-like image is stored in the memory 38 of the image processing unit 31 in advance. The CPU 36 processes images for changing the graphic image data such that the width of the belt of the standard belt image is increased or decreased the overlap width of the belt is changed, or the extending range of the belt is changed respectively in accordance with the values of the repetition rate, the intensity and the irradiation range calculated from the inputted data, whereby the belt-like graphic image as in Fig. 1 is generated. The generated graphic image data is inputted into the display memory 37.

When a scanogram is taken at the beginning, an image of the scanogram is generated from data from the detector 2 in the image reconstruction circuit 35, while the positional information of the subject 10 is detected. The image data of the scanogram 10' is inputted into the display memory 37. The image data is superimposed accurately on the belt-like graphic image of the irradiation range from the CPU on the basis of the positional information of the scanogram 10' and the information of the set slice position, wherein data of an image as shown in Fig. 1 is obtainable. This image data is inputted to the display monitor 34. The X-ray scanning conditions thus inputted are reflected on the belt-like lines 11 on the monitor 34 immediately. Therefore, the operator can set optimum scanning conditions in the slice position while confirming the X-ray irradiation range, the X-ray repetition rate, the X-ray intensity and so on.

Display Example 1: (an example of increasing the number of image reconstruction positions while confirming the X-ray irradiation range)

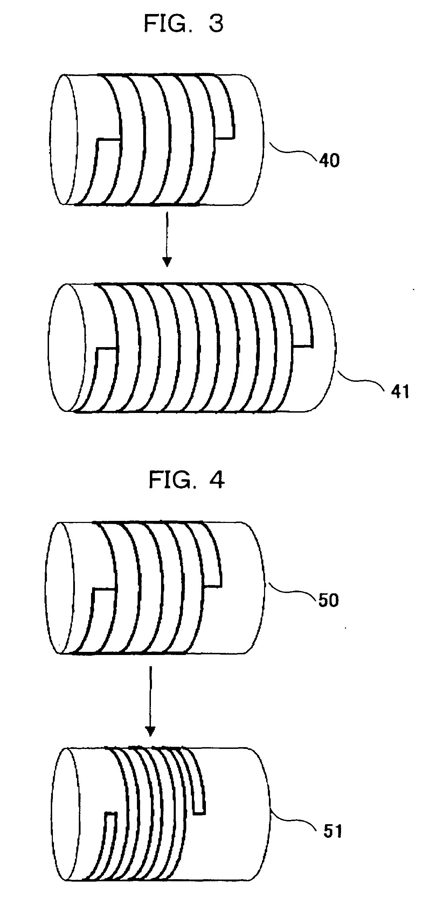

The number of image reconstruction positions or the number of scans is increased using the input unit like the keyboard 32, or the mouse 33. When the number of image reconstruction positions or the number of scans is increased, the X-ray irradiation range changes as in Fig. 3. Compared with the X-ray irradiation range before the change represented by the reference numeral 40 in Fig. 3, the X-ray irradiation range after the change represented by the reference numeral 41 remains the same and only the number of belt-like lines increases, whereby the X-ray irradiation range expands. The X-ray repetition rates shows no change irrespective of the change of the X-ray irradiation range.

Display Example 2: (an example of reducing the X-ray irradiation width while confirming the X-ray repetition rate)

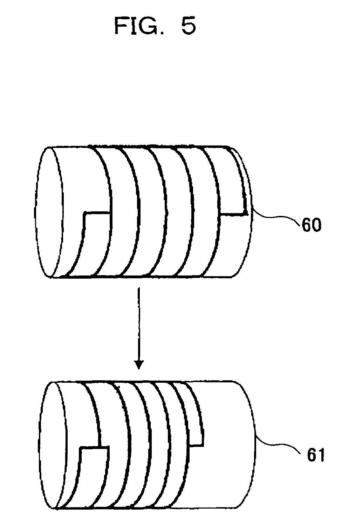

The X-ray irradiation width is reduced using the input unit like the keyboard 32 or the mouse 33. When the X-ray irradiation width is reduced, the belt-like lines having a width corresponding to the scanogram image size and the X-ray irradiation width are displayed as shown in Fig. 4. Compared with the X-ray irradiation width before the change represented by the reference numeral 50 in Fig. 4, the X-ray irradiation width after the change represented by the reference numeral 51 in Fig. 4 is reduced, and both the X-ray repetition rate and the X-ray irradiation range are reduced.

Display Example 3: (an example for reducing the table feed pitch while confirming the X-ray repetition rate)

The table feed pitch is reduced using the input unit like the keyboard 32 or the mouse 33. By changing the table feed pitch, the belt-like lines are displayed at intervals corresponding to the scanogram image size and the table feed pitch as in Fig. 5. Compared with the table feed pitch before the change represented by the reference numeral 60, in the table feed pitch after the change represented by the reference numeral 61, the irradiation width remains the same and the X-ray repetition rate is increased, whereby the X-ray irradiation range is reduced.

Display Example 4: (an example to increase the tube voltage or the tube current while confirming the X-ray intensity)

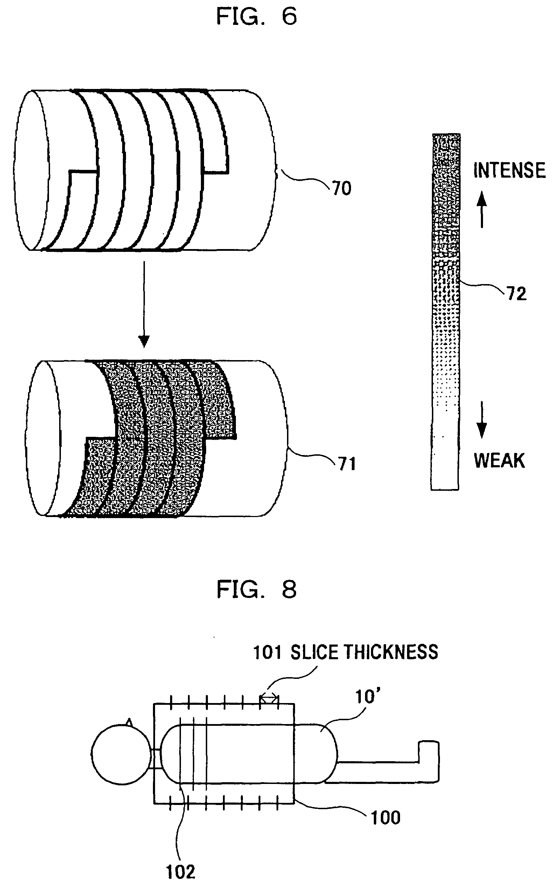

The change of the tube voltage or the tube current is selected, and the tube voltage or the tube current is increased in use of the input unit like the keyboard 32 or the mouse 33. When the tube voltage or the tube current is changed, the belt-like lines are displayed in a color or a contrast corresponding to the tube voltage or the tube current as shown in Fig. 6.

Compared with the display color of the belt-like lines before change represented by the reference numeral 70 in Fig. 6, the belt-like lines after change represented by the reference numeral 71 in Fig. 6 are displayed in an emphatic color. If necessary, a bar graph showing a relationship between the X-ray intensity and the contrast, represented by the reference numeral 72 in Fig. 6, is displayed on the display 34 so that the X-ray intensity can be intuitively and easily understood. Alternatively, the numeric value of the X-ray intensity may be displayed.

The color of the belt indicating the irradiated portion is determined by parameters such as the scanner rotational speed (time per revolution), the tube current, the tube voltage, the repetition rate, and the like. In a case where a function capable of changing the tube current or the scanner rotational speed in accordance with a portion to be scanned during the measurement of a CT image, the belt can be displayed in a color changed in response to the portion.

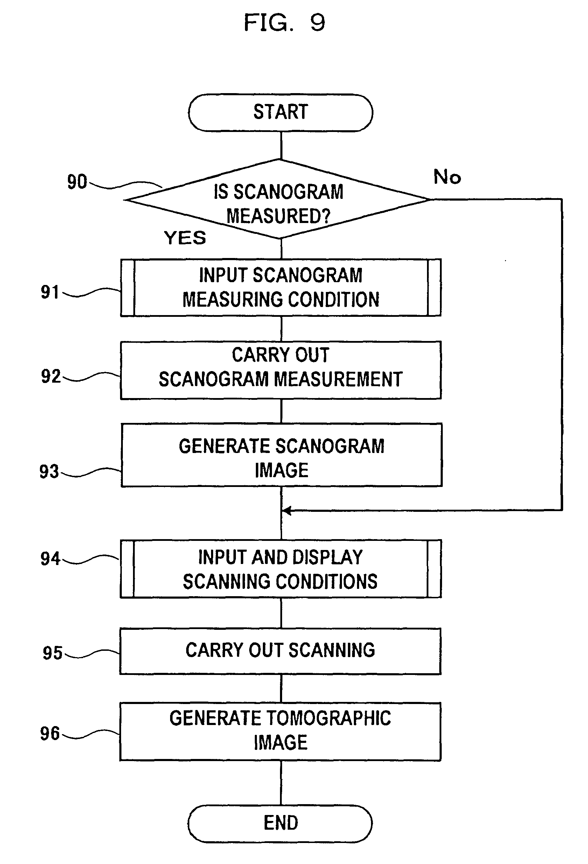

Further, another displaying example different from the belt-like display of the graphic image of the X-ray irradiation range is shown in Fig. 8. The X-ray irradiation range may be displayed by a rectangular frame 100 on the scanogram 10' of the subject while a scale indicating the slice thickness is displayed on the frame 100. The slice positions may be displayed by lines like the reference numeral 102.

Next, the procedure from the step of measuring a scanogram to the step of finally obtaining a CT image by the X-ray CT device according to the present invention will be described with reference to the flow chart of Fig. 9.

In Step 90, it is judged whether or not a scanogram of a subject to be examined is measured. In Step 91, scanogram measuring conditions are inputted. Here, the X-ray tube current and the X-ray tube voltage are set in accordance with a portion of a body to be measured. In Step 92, the measurement of the scanogram is carried out. Next, in Step 93, an image data of a scanogram image is generated from the measured data in the image reconstruction circuit 35. Next, in Step 94, scanning conditions are inputted. Based on the inputted conditions, the CPU 36 calculates the repetition rate, the intensity, the irradiation range and so on, generates a graphic image, and displays it on the display while superimposing it on the scanogram. While viewing the display image, the operator inputs various different conditions in consideration of the physique of the subject, the intended use of the image, the location to be measured, and so on, visually confirms the change of the graphic image as to the repetition rate, the intensity, the irradiation range and the like, and determines optimum scanning conditions. Next, in Step 95, the scanning conditions determined in Step 94 are set, and scanning is carried out at a desired slice location. In Step 96, a CT image is generated from data obtained by the detector 2 in the image reconstruction circuit 35, and displayed.

The invention discovered by the present inventor has been specifically described based on its embodiment. However, the present invention is not limited to the embodiment, and various modification can be made without departing its spirit and scope.

Industrial Applicability

Effects obtained by representatives of the invention disclosed herein will be described briefly.

According to the present invention, X-ray irradiation conditions such as the X-ray irradiation range, the X-ray repetition rate, and the X-ray intensity can be intuitively understood on the monitor. Accordingly, the X-ray scanning conditions can be easily confirmed so that the operability of the X-ray CT device can be improved.

In addition, since the X-ray scanning conditions can be easily confirmed, reduction of erroneous operations can be expected, whereby reduction in ineffective exposed dose can be expected.

**Claims**

1. An X-ray CT device comprising:

   an X-ray source;
   a detector for detecting X-rays radiated from said X-ray source and transmitted through a subject to be examined;

   an image reconstruction unit for processing X-ray data detected by said detector to thereby generate a tomographic image of said subject;
   a display unit for displaying said tomographic image;
   a memory unit for storing said data obtained by said X-ray detector and data of said tomographic image;
   an input unit for inputting data of tomographic scanning conditions of said subject; and
   an image processor for calculating an X-ray repetition rate, an X-ray intensity and an X-ray irradiation range in said subject based on said inputted data of scanning conditions, and generating image data for displaying at least a value of any one of said X-ray repetition rate, said X-ray intensity and said X-ray irradiation range as a graphic image on said display unit based on the result of said calculation.

2. An X-ray CT device according to Claim 1, wherein said graphic image of said X-ray irradiation range is expressed by a belt-like figure.

3. An X-ray CT device according to Claim 1, wherein said graphic image of said X-ray repetition rate is expressed by an overlap of a belt-like figure.

4. An X-ray CT device according to Claim 1, wherein said graphic image of said X-ray intensity is expressed by a difference in color or that in brightness of a belt-like figure.

5. An X-ray CT device according to Claim 1, further comprising:

   a scanner unit in which said X-ray source and said detector are oppositely disposed and interposing said subject therebetween, and said X-ray source and said detector revolve around said subject while radiating X-rays to said subject;

   wherein said subject further relatively moves in a direction crossing a direction of said radiation with respect to said scanner unit.

6. An X-ray CT device according to Claim 5, wherein said data of scanning conditions for calculating said X-ray repetition rate includes at least a slice thickness of said subject and a moving speed of said subject.

7. An X-ray CT device according to Claim 5, wherein said data of scanning conditions for calculating said X-ray intensity includes at least a rotational speed of said X-ray source and said detector in rotating around said subject, and a voltage value and a current value of said X-ray source.

8. An X-ray CT device according to Claim 1, further comprising:

   means for generating a scanogram image of said subject or a subject image imitative of said subject;

   wherein said image processor includes means for generating an image obtained by superimposing said scanogram image or said subject image on said graphic image.

9. An X-ray CT device according to Claim 8, wherein said graphic image of said X-ray irradiation range is displayed on said scanogram image or said subject image in a form of a frame figure corresponding to said irradiation range, and a scale indicating the slice thickness is further displayed on said frame figure.

10. An X-ray CT device according to Claim 1, wherein said image processor makes said display unit display at least a numeric value of any one of said calculated X-ray repetition rate, X-ray intensity and X-ray irradiation range together with said graphic image.

11. An image processor of an X-ray CT device, comprising:

    an input unit for inputting data obtained by detecting X-rays radiated from an X-ray source and transmitted through a subject to be examined, and data of tomographic scanning conditions of said subject;
    a memory unit for storing data of said tomographic image; and
    an image processing portion for calculating an X-ray repetition rate, an X-ray intensity and an X-ray irradiation range in said subject based on said inputted data of scanning conditions, and generating image data for displaying at least a value of any one of said X-ray repetition rate, said X-ray intensity and said X-ray irradiation range as a graphic image on a display unit based on a result of said calculation.

12. An image processor according to Claim 11, wherein said graphic image of said X-ray irradiation range is expressed by a belt-like figure.

13. An image processor according to Claim 11, wherein said graphic image of said X-ray repetition rate is expressed by an overlap of said belt-like figure.

14. An image processor according to Claim 11, wherein said graphic image of said X-ray intensity is expressed by a difference in color or brightness of a belt-like figure.

15. An image processor according to Claim 11, further comprising:

    means for generating an image superimposing a scanogram image of said subject or a subject image imitative of said subject on said graphic image.

16. An image processor according to Claim 15, wherein said graphic image of said X-ray irradiation range is displayed on said scanogram image or said subject image in a form of a frame figure corresponding to said irradiation range, and a scale indicating a slice thickness is further displayed on said frame figure.

17. An image processor according to Claim 11, wherein said data of scanning conditions for calculating said X-ray repetition rate includes at least a slice thickness of said subject and a moving velocity of said subject.

18. An image processor according to Claim 11, wherein said data of scanning conditions for calculating said X-ray intensity includes at least an orbital speed for said X-ray source and said detector to revolve around said subject, and a voltage value and a current value of said X-ray source.

19. An image processor according to Claim 11, wherein said image processing portion makes said display unit display at least a numeric value of any one of said calculated X-ray repetition rate, X-ray intensity and X-ray irradiation range together with said graphic image.

20. An image processing method for making an X-ray CT device generate an image of a subject to be examined, comprising the steps of:

    receiving data of scanning conditions specified for obtaining a tomographic image of said subject in said X-ray CT device;
    calculating an X-ray repetition rate, an X-ray intensity and an X-ray irradiation range in said subject based on said inputted data of scanning conditions;
    generating image data for displaying at least a value of any one of said X-ray repetition rate, said X-ray intensity and said X-ray irradiation range as a graphic image on a display unit based on a result of said calculation;
    determining and inputting setting conditions of tomographic scanning of said subject based on said image displayed on said display unit; and performing tomographic scanning upon said subject in said X-ray CT device based on said determined scanning conditions.

21. An image processing method according to Claim 20, wherein said step of generating said image data includes the step of expressing said graphic image of said X-ray irradiation range by a belt-like figure.

22. An image processing method according to Claim 20, wherein said step of generating said image data includes the step of expressing said graphic image of said X-ray repetition rate by an overlap of said belt-like figure.

23. An image processing method according to Claim 20, wherein said step of generating said image data includes the step of expressing said graphic image of said X-ray intensity by a difference in color or brightness of a belt-like figure.

24. An image processing method according to Claim 20, wherein said calculating step includes the step of making a calculation using at least a slice thickness of said subject and a moving velocity of said subject as said data of scanning conditions for calculating said X-ray repetition rate.

25. An image processing method according to Claim 20, wherein said calculating step includes the step of making a calculation using at least an orbital speed for said X-ray source and said detector to revolve around said subject, and a voltage value and a current value of said X-ray source as said data of scanning conditions for calculating said X-ray intensity.

26. An image processing method for making an X-ray CT device generate an image of a subject to be examined, comprising the steps of:

generating a scanogram of said subject in said X-ray CT device;
receiving data of scanning conditions specified for obtaining a tomographic image of said subject;
calculating an X-ray repetition rate, an X-ray intensity and an X-ray irradiation range in said subject based on said inputted data of scanning conditions;
generating image data for displaying at least a value of any one of said X-ray repetition rate, said X-ray intensity and said X-ray irradiation range as a graphic image on a display unit based on a result of said calculation;
displaying said scanogram and said graphic image superimposed on each other on said display unit;
determining and inputting setting conditions of tomographic scanning of said subject based on said image displayed on said display unit; and performing tomographic scanning upon said subject in said X-ray CT device based on said determined scanning conditions.

27. An image processing method according to Claim 26, wherein said step of generating said image data includes the step of expressing said graphic image of said X-ray irradiation range by a belt-like figure.

28. An image processing method according to Claim 26, wherein said step of generating said image data includes the step of expressing said graphic image of said X-ray repetition rate by an overlap of said belt-like figure.

29. An image processing method according to Claim 26, wherein said step of generating said image data includes the step of expressing said graphic image of said X-ray intensity by a difference in color or brightness of a belt-like figure.

30. An image processing method according to Claim 26, wherein said step of generating said image data includes the step of displaying said graphic image of said X-ray irradiation range on top of said scanogram in a form of a frame figure corresponding to said irradiation range, and further displaying a scale indicating a slice thickness on said frame figure.

31. An image processing method according to Claim 26, wherein said calculating step includes the step of making a calculation using at least a slice thickness of said subject and a moving velocity of said subject as said data of scanning conditions for calculating said X-ray repetition rate.

32. An image processing method according to Claim 26, wherein said calculating step includes the step of making a calculation using at least an orbital speed for said X-ray source and said detector to revolve around said subject, and a voltage value and a current value of said X-ray source as said data of scanning conditions for calculating said X-ray intensity.

FIG. 1

ENLARGED VIEW

10'

11

12

# FIG. 2

30

1

10

5

4

2

31 IMAGE PROCESSING UNIT

SCANOGRAM IMAGE DATA

37

34

MEASURED DATA

IMAGE RECONSTRUCTION CIRCUIT

35

DISPLAY MEMORY

DISPLAY

36 — CPU

38 — MEMORY

GRAPHIC IMAGE DATA

SCANNING CONDITIONS SUCH AS SLICE, AND TUBE CURRENT

KEYBOARD

MOUSE

32

33

FIG. 3

FIG. 4

FIG. 5

## FIG. 6

INTENSE

70

72

71

WEAK

## FIG. 8

101 SLICE THICKNESS

10'

100

102

# FIG. 7

ENLARGED
VIEW

FIG. 9

```
              ┌─────────────────┐
              │      START      │
              └─────────────────┘
                       │
        90             ▼
          ╱──────────────────────────╲      No
         ╱      IS SCANOGRAM           ╲──────────┐
         ╲        MEASURED?            ╱          │
          ╲──────────────────────────╱           │
       YES           │                            │
              ┌─────────────────┐                 │
       91     │ INPUT SCANOGRAM │                 │
              │MEASURING CONDITION│               │
              └─────────────────┘                 │
                       │                          │
              ┌─────────────────┐                 │
       92     │    CARRY OUT    │                 │
              │SCANOGRAM MEASUREMENT│             │
              └─────────────────┘                 │
                       │                          │
              ┌─────────────────┐                 │
       93     │GENERATE SCANOGRAM│                │
              │      IMAGE      │                 │
              └─────────────────┘                 │
                       │◄─────────────────────────┘
                       ▼
              ┌─────────────────┐
       94     │ INPUT AND DISPLAY│
              │SCANNING CONDITIONS│
              └─────────────────┘
                       │
              ┌─────────────────┐
       95     │ CARRY OUT SCANNING│
              └─────────────────┘
                       │
              ┌─────────────────┐
       96     │GENERATE TOMOGRAPHIC│
              │      IMAGE      │
              └─────────────────┘
                       │
              ┌─────────────────┐
              │      END        │
              └─────────────────┘
```

## INTERNATIONAL SEARCH REPORT

| International application No. |
| --- |
| PCT/JP02/05284 |

| A. CLASSIFICATION OF SUBJECT MATTER |
| --- |
| Int.Cl$^7$ A61B6/03 |
| According to International Patent Classification (IPC) or to both national classification and IPC |

| B. FIELDS SEARCHED |
| --- |
| Minimum documentation searched (classification system followed by classification symbols) |
| Int.Cl$^7$ A61B6/00-6/14 |

Documentation searched other than minimum documentation to the extent that such documents are included in the fields searched

| | | | |
| --- | --- | --- | --- |
| Jitsuyo Shinan Koho | 1922-1996 | Toroku Jitsuyo Shinan Koho | 1994-2002 |
| Kokai Jitsuyo Shinan Koho | 1971-2002 | Jitsuyo Shinan Toroku Koho | 1996-2002 |

Electronic data base consulted during the international search (name of data base and, where practicable, search terms used)

| C. DOCUMENTS CONSIDERED TO BE RELEVANT | | |
| --- | --- | --- |
| Category* | Citation of document, with indication, where appropriate, of the relevant passages | Relevant to claim No. |
| Y | JP 8-215189 A (Toshiba Medical Engineering Kabushiki Kaisha), 27 August, 1996 (27.08.96), Full text; Figs. 1 to 7 (Family: none) | 1,2,4,5,7-12,14-16,18-21,23,25-27,29,30,32 |
| A | Full text; Figs. 1 to 7 (Family: none) | 3,6,13,17,22,24,28,31 |
| Y | JP 7-204196 A (Hitachi Medical Corp.), 08 August, 1995 (08.08.95), Full text; Figs. 1 to 4 (Family: none) Full text; Figs. 1 to 4 (Family: none) | 1,2,4,5,7-12,14-16,18-21,23,25-27,29,30,32 |
| A | | 3,6,13,17,22,24,28,31 |
| Y | JP 2000-116634 A (Toshiba Corp.), 25 April, 2000 (25.04.00), Full text; Figs. 1 to 5 (Family: none) | 7,18,25,32 |

| ☐ Further documents are listed in the continuation of Box C. | ☐ See patent family annex. |
| --- | --- |

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
| --- | --- |
| 21 June, 2002 (21.06.02) | 02 July, 2002 (02.07.02) |

| Name and mailing address of the ISA/ | Authorized officer |
| --- | --- |
| Japanese Patent Office | |
| Facsimile No. | Telephone No. |

Form PCT/ISA/210 (second sheet) (July 1998)